# EUROPÄISCHE PATENTSCHRIFT

(11) **EP 1 035 130 B1**
(45) Veröffentlichungstag und Bekanntmachung des Hinweises auf die Patenterteilung: **09.10.2002**
(21) Anmeldenummer: 00104418.9
(22) Anmeldetag: 03.03.2000
(51) Int. Cl.: C07K 1/12

(54) **Trennverfahren zum Lösen von linkergekoppelten Substanzen von einer Polymeroberfläche**
Process of desorption of linker-bound substances from a polymeric surface
Procédé de dissolution de substances liées par un linker d'une surface polymérique

(30) Priorität: 04.03.1999 DE 19909584
(43) Veröffentlichungstag der Anmeldung: 13.09.2000
(73) Patentinhaber: HaemoSys GmbH, 07747 Jena (DE)
(72) Erfinder: Götz, Nowak, Prof. Dr., 99097 Erfurt (DE); Bucha, Elke, Dr., 99094 Erfurt (DE)
(74) Vertreter: VOSSIUS & PARTNER

(56) Entgegenhaltungen:
- DE-A- 19 715 504

## Beschreibung

Die vorliegende Erfindung betrifft ein Verfahren zum Lösen von linkergekoppelten Substanzen von einer Polymeroberfläche, in dem die Bindung des Linkers zur Oberfläche mittels eines oder mehrerer polarer organischer Lösungsmittel getrennt wird, wobei die Linker gemäß der vorliegenden Erfindung mindestens ein zu einer Wasserstoffbrückenbindung fähiges Strukturelement aufweisen. Polymere, auf deren Oberfläche die erfindungsgemäß zu lösende Bindung auftritt, umfassen, bevorzugt in Seitenketten des Polymergrundgerüsts, Carbonylgruppen in Form von Ketogruppen oder Carbonsäuregruppen oder ihren Derivaten.

Das Prinzip der Interaktion bestimmter Linker mit solchen Polymeroberflächen ist in WO 98/46648 beschrieben. Es kann beispielsweise dazu verwendet werden, durch Einsatz linkermodifizierter Oberflächen und/oder linkermodifizierter Wirksubstanzen eine Entfernung von Wirksubstanzen aus Flüssigkeiten zu erreichen, oder aber solche Substanzen in Flüssigkeiten zu präsentieren. Die genannte Anmeldung offenbart weiterhin die Anwendung dieses Interaktionsprinzips im Rahmen umfangreicher medizinischer Verfahren. So können mittels eines Adsorptionssystems bestehend aus einer Polymeroberfläche und einem damit verbundenen Linker der spezifische funktionelle Gruppen aufweist, Wirkstoffe aus Körperflüssigkeiten entfernt werden, die mit dem im Linker vorhandenen funktionellen Gruppen Bindungen eingehen. Weiterhin erlaubt ein solches System das gezielte Einbringen von Wirksubstanzen in den Körper eines Patienten, ohne daß es zu einer weiträumigen Ausbreitung der Substanz im Körper kommt, die zu möglichen Komplikationen und Nebenwirkungen führen könnte. Auch erlaubt das in der Anmeldung WO 98/46648 beschriebene Kopplungsprinzip eine Modifikation von Oberflächen, die mit physiologischen Systemen in Berührung kommen, wie z.B. Prothesen, Filter für physiologische Flüssigkeiten oder Dialysegeräte. Die Linker, die mindestens ein zur Wasserstoffbrückenbindung fähiges Strukturelement aufweisen, zeigen nach Aufbringen auf die genannten Oberflächen eine erstaunlich hohe Bindungsstärke, so daß sie weder durch Temperaturerhöhung, z.B. auf bis zu 70°C noch durch Spülen mit wäßrigen Lösungen wieder von diesen Oberflächen zu entfernen waren, selbst wenn die Lösungen hohe Ionenstärken besaßen, wie z.B. 2n Glycin oder 2n Harnstoff-Lösungen, oder pH-Werte zwischen 2 und 13 aufwiesen.

Im Zusammenhang mit bestimmten Anwendungen wäre jedoch die Möglichkeit einer schonenden Ablösung der linkergekoppelten Substanzen von der Oberflächenmatrix wünschenswert. Dies gilt z.B. in solchen Fällen, wo eine Analyse der Substanzen nach ihrer Kopplung mit dem Linker und der Entfernung aus der entsprechenden Flüssigkeit für diagnostische oder Forschungszwecke angestrebt wird. Weiter wäre eine Trennung in solchen Fällen vonnöten, wo vor der erneuten Nutzung der Oberfläche oder der linkergekoppelten Substanz eine oder beide Komponenten sterilisiert werden können, um anschließend einzeln oder gemeinsam wiederverwendet zu werden.

Der vorliegenden Erfindung liegt daher die Aufgabe zugrunde, ein schonendes, zerstörungsfreies und dabei einfaches und kostengünstiges Verfahren bereitzustellen, mit dem die Trennung eines Systems aus einer Oberfläche und durch Linker daran gekoppelten Substanzen bereitzustellen, um so die Wiederverwertbarkeit aller Komponenten zu sichern. Diese Aufgabe wird erfindungsgemäß gelöst, indem das System einem polaren organischen Lösungsmittel ausgewählt aus Alkanolen und Estern ausgesetzt wird. Überraschenderweise wurde gefunden, daß solche Lösungsmittel auch in Form wäßriger Lösungen geringer Konzentration fähig sind, die Bindung zwischen der linkergekoppelten Substanz und der Polymeroberfläche vollständig zu lösen. Sowohl die Substanzen als auch die Oberflächen behalten nach Lösung der Bindung ihre Funktionsfähigkeit.

Polymere, von denen Linker mit den daran gekoppelten Substanzen in erfindungsgemäßer Form gelöst werden können, sind beispielsweise in WO 98/46648 beschrieben. Zum Einsatz kommen Homo- oder Copolymere, zu deren Herstellung mindestens ein Monomertyp eingesetzt wird, der neben einer polymerisierbaren Doppelbindung oder einer polykondensierbaren funktionellen Gruppe eine weitere Carbonylgruppe in Form eines Ketons oder eines Carbonsäurederivats enthält, die nicht an der Polymerisationsreaktion teilnimmt. Bevorzugt enthält das Polymer ein Strukturelement der Formel (A) : 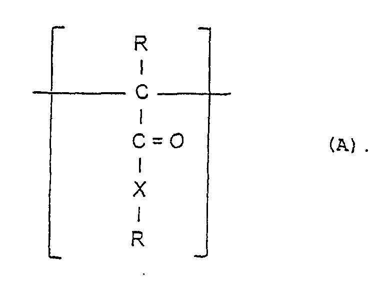 wobei die Reste R gleich oder verschieden sein können und einen Alkyl oder Arylrest oder ein Wasserstoffatom darstellen. Der Alkylrest kann linear oder verzweigt sein und besteht bevorzugt aus 1 bis 20 Kohlenstoffatomen. Der Arylrest besteht bevorzugt aus 6 bis 18, besonders bevorzugt aus 6 bis 12 Kohlenstoffatomen. Der Rest X ist fakultativ und bedeutet 0, N oder CH₂. Für den Fall X = N trägt N zusätzlich zu dem in Formel (A) vermerkten einen weiteren Rest R, der unabhängig von den anderen Resten R wie vorstehend definiert ist.

Besonders bevorzugt als Alkylrest ist ein geradkettiger oder verzweigter, gegebenenfalls substituierter C₁₋8-Alkylrest, beispielsweise ein Methyl-, Ethyl- oder Propylrest. Beispiele für gegebenenfalls vorhandene Substituenten umfassen ein oder mehrere Halogenatome, z.B. Fluor-, Chlor-, Brom- oder Iodatome oder Hydroxylgruppen, C₁₋₆-Alkylreste oder C₁₋₆-Alkoxyreste oder C₁₋₆-Alkylthiolreste. Der Arylrest ist besonders bevorzugt ein monocyclischer oder bicyclischer, gegebenenfalls substituierter Arylrest, der gegebenenfalls ein oder mehrere Heteroatome enthalten kann. Beispiele für solche Arylreste sind Phenyl, 1- oder 2-Naphthyl, Indenyl- oder Isoindenylreste. Beispiele für heteroatomhaltige Arylreste sind C₃₋₉-Heteroarylreste, die Heteroatome, ausgewählt aus Sauerstoff-, Schwefeloder Stickstoffatomen, enthalten. Monocyclische Heteroarylreste umfassen beispielsweise Pyrolyl-, Furyl-, Thienyl-, Imidazolyl-, N-Methylimidazolyl-, N-Ethylimidazolyl-, Benzothiazolyl-, Chinazolinyl-, Naphthylpyridinyl-, Chinolinyl-, Isochinolinyl- und Tetrazolylreste.

Ein bevorzugtes Polymer, das solche Gruppen enthält, ist ein Polyalkylmethacrylat (PAMA), wie z.B. Polymethylmethacrylat (PMMA), Polyethylmethacrylat (PEMA) oder Polypropylmethacrylat. Besonders bevorzugt aus dieser Gruppe wird Polymethylmethacrylat eingesetzt. Weiterhin können Polyvinylacetat, Polycyclohexylmethacrylate oder Polyphenylmethacrylat eingesetzt werden. Weiterhin können Copolymere aus beliebigen Anteilen der vorstehend genannten Polymere untereinander und/oder mit einer oder mehrerer weiterer Polymerkomponenten, beispielsweise Polystyrol, Polyacrylnitril oder Polyamiden eingesetzt werden. Bevorzugt beträgt der Anteil des Carbonylfunktionellen Monomers, z.B. Alkylmethacrylat, an solchen Mischpolymeren mindestens 20 mol-%, besonders bevorzugt 40 mol-% und ganz besonders bevorzugt 60 mol-%.

Die Form der eingesetzten Polymeroberflächen ist durch das Prinzip der vorliegenden Erfindung nicht beschränkt. Es können beispielsweise Polymere in Form von Folien, Hohlkörpern wie z.B. Schläuche, Membranen oder Mikropartikeln eingesetzt werden.

Substanzen, die vor der Trennung der entsprechenden Bindung mittels eines Linkers mit der Polymeroberfläche gekoppelt werden, können je nach Anwendung des Systems ausgewählt werden. Bevorzugt werden pharmakologisch oder physiologisch aktive Wirkstoffe eingesetzt. Beispiele hierfür sind Proteine, Nucleinsäuren oder zelluläre Signalstoffe. Besonders bevorzugt ist als Protein ein Enzym, ein Antigen, ein Antikörper, ein Tumormarker, ein Oberflächenantigen, ein Ligand, ein Rezeptor, ein oberflächenaktives Zellfragment von Bakterien oder Viren oder ein Immunbotenstoff. Die pharmakologisch wirksame Substanz ist beispielsweise ein Gerinnungshemmstoff, ein stoffwechselaktives Enzym, oder ein synthetisches Arzneimittel, wie beispielsweise ein Antibiotikum, ein Antitumormittel oder ein Enzyminhibitor. Substanzen, die für das vorliegende System eingesetzt werden, sind ausführlich in WO 98/46648 beschrieben.

Linker, deren Bindung mit einer spezifischen Polymeroberfläche mit dem offenbarten Verfahren gelöst werden kann, sind Moleküle, die mindestens zwei funktionelle Gruppen L1 und L2 aufweisen. Eine dieser funktionellen Gruppen (L1) muß zur Bildung von Wasserstoffbrücken fähig sein und so die Bindung des Linkers an die Polymeroberfläche ermöglichen. Die funktionelle Gruppe L2 wird so gewählt, daß eine Bindung zwischen dem Linker und der zu koppelnden Substanz hergestellt werden kann. Um mehrere Substanzen gleichzeitig auf der Polymeroberfläche aufbringen zu können, ist die gleichzeitige Verwendung mehrerer Linker mit unterschiedlichen Gruppen L2 möglich. Jedoch besteht auch die Möglichkeit, Linker eines Typs einzusetzen, die mehrere Gruppen L2 besitzen, die gleich oder unterschiedlich sind. Gleichermaßen können auch Linker eingesetzt werden, die mehrere gleiche oder unterschiedliche Gruppen L1 aufweisen. Bevorzugt sind L1 und L2 durch eine Alkylkette oder einen Polyether von 1 bis 20 Kohlenstoffatomen verbunden.

Strukturelement L1 ist bevorzugt ein polares Wasserstoffatom, wie es beispielsweise in OH-, SH-, NH- oder PH-Bindungen vorliegt. Dieses Strukturelement befindet sich bevorzugt an einer ausreichend wasserlöslichen Verbindung als Linker, der weiter das Strukturelement L2 trägt. Besonders bevorzugt ist L1 endständig am Linker angebracht.

Die funktionelle Gruppe, mittels derer die Substanz an den Linker gebunden werden kann (L2) ist beispielsweise ein Succinimidylsuccinat, Succinimidylpropionat, Nitrophenylcarbonat, Trisylat, Epoxid, Aldehyd, Isocyanat oder ein Maleinimid.

Funktionelle Gruppen L2, mittels derer die bevorzugten Linker zur Anbindung einer Substanz modifiziert werden können, sind z.B. im Katalog der Firma Shearwater Polymers, Inc., 2307 Spring Branch Rd., Huntsville, AL 35801 (USA) beschrieben.

Bevorzugt werden als Linker Polyalkylenglykole eingesetzt, besonders bevorzugt Polyethylenglykol (PEG). Weiter bevorzugt sind Polyalkylenimine, Polyalkylenamine oder Polyalkylensulfide, sowie Polyoxaziline. Insbesondere bevorzugt werden Polyethylenglycole eingesetzt. Die genannten Verbindungen besitzen vorzugsweise ein Molekulargewicht von 5-50 kDa.

Polare organische Lösungsmittel im Sinne der vorliegenden Erfindung sind Alkanole und Ester, deren Moleküle eine polare kovalente Bindung aufweisen, die dem Molekül ein elektrisches Dipolmoment verleiht. Die Lösungsmittelmoleküle der im Rahmen der Erfindung einzusetzenden Lösungsmittel besitzen bevorzugt ein elektrisches Dipolmoment in der Gasphase, das größer ist als 1,0 D, besonders bevorzugt größer als 1,4 D und ganz besonders bevorzugt größer als 1,6 D.

Bevorzugt werden Alkanole eingesetzt, die bis zu 3 Kohlenstoffatome und bis zu 3 Hydroxylgruppen tragen. Besonders bevorzugt ist Methanol.

Die organischen Lösungsmittel werden bevorzugt in Form einer wäßrigen Lösung eingesetzt, deren Lösungsmittelkonzentration bevorzugt kleiner als 70 Volumen-% ist und besonders bevorzugt zwischen 10 und 50 Vol.-% liegt.

Gegebenenfalls ist das Lösungsmittel in Abhängigkeit von der an den Linker gebundenen Substanz so auszuwählen, daß keine irreversible Reaktion der Substanz mit dem Lösungsmittel auftreten kann.

Die Trennung der linkergekoppelten Substanz von der Oberfläche kann sowohl durch Inkubation der linkerbehafteten Oberfläche im Lösungsmittel, durch Spülung der Oberfläche oder mittels eines Durchströmungsverfahrens erfolgen.

Anhand des folgenden Beispiels soll die Erfindung illustriert werden:

### Beispiel

### Lösung der PEG-Hirudin-Bindung an Polymethylmethacrylat (PMMA)-Partikel

Poröse PMMA-Partikel werden in zwei Ansätzen mit PEG-Hirudin beladen. Im Anschluß daran erfolgt ein 10minütiger Spülvorgang mit unterschiedlich konzentrierter Methanollösung bzw. in Vergleichsuntersuchungen mit physiologischer Kochsalzlösung (NaCl). Die Spüllösungen werden auf abgelöstes PEG-Hirudin mittels biologischem Funktionsnachweis untersucht und diese wiedergewonnen.

Im Anschluß an die Spülvorgänge der Partikel wird ihre Beladungskapazität durch erneutes Beladen mittels PEG-Hirudin-Lösungen geprüft. Das aus den Methanol-Spüllösungen rückgewonnene PEG-Hirudin wird ebenfalls auf eine erneute Bindungsfähigkeit zu PMMA-Partikeln getestet.

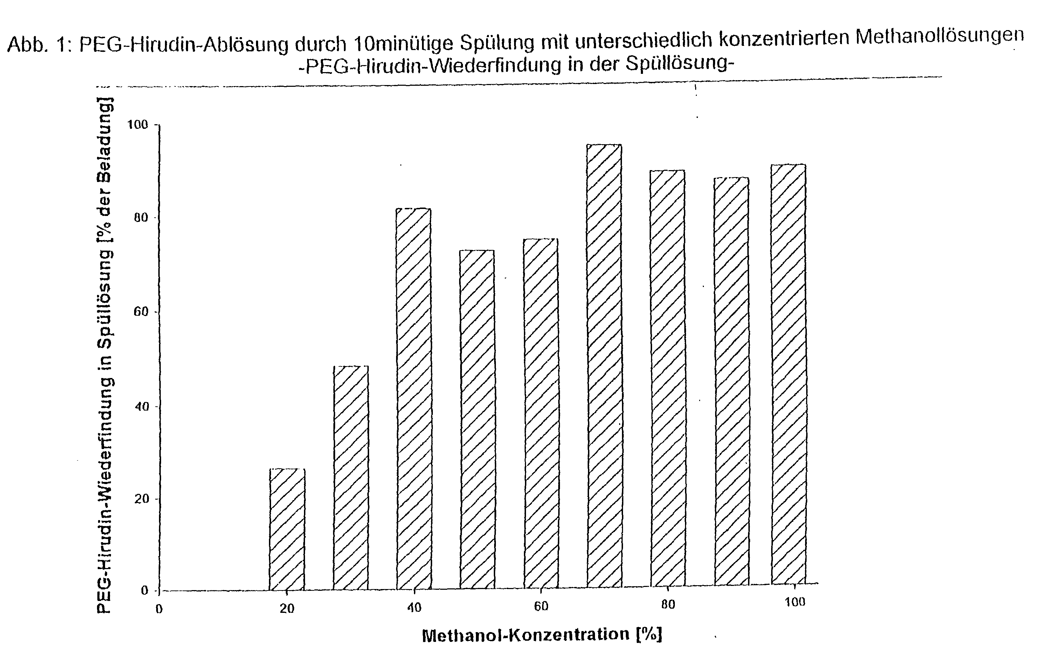

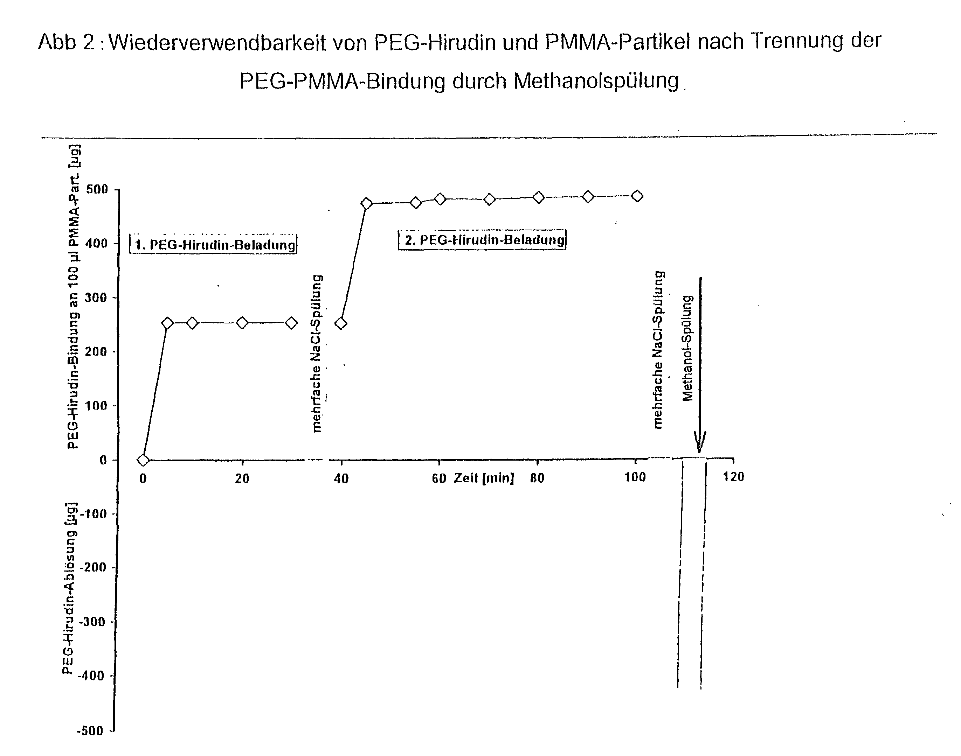

Mittels Verdünnungen bis zu einer Methanol-Konzentration von 40 % konnten während einer nur 10minütigen Spülung mehr als 80 % des oberflächengebundenen PEG-Hirudins abgelöst und zurückgewonnen werden (Tab. 1; Abb. 1).
Die Wiederverwendbarkeit sowohl gereinigter Partikel als auch rückgewonnenen PEG-Hirudins demonstriert Abbildung 2. Jeweils 50,5 Mio. unbenutzte bzw. gereinigte Partikel zeigten nahezu die gleiche Bindungskapazität für PEG-Hirudin. Ebenfalls dargestellt ist das gleiche Bindungsverhalten von unbenutztem und als Methanol-Spüllösung rückgewonnenen PEG-Hirudins.

In allen Versuchen war das oberflächenfixierte PEG-Hirudin funktionell aktiv, was durch die Prüfung des Thrombinhemmvermögens belegt wurde.

**Tab. 1**

| Methanol-Konzentration [%] | PEG-Hirudin-gebunden an PMMA-Partikel [µg] | PEG-Hirudinrückgewonnen aus Methanol-Spüllösung [µg] |
|---|---|---|
| 99,9 | 485,0 | 436,2 |
| 90 | 464,5 | 399,4 |
| 80 | 457,5 | 438,0 |
| 70 | 491,4 | 468,3 |
| 60 | 530,2 | 397,0 |
| 50 | 511,6 | 371,3 |
| 40 | 527,0 | 430,3 |
| 30 | 538,2 | 261,0 |
| 20 | 509,8 | 135,0 |
| 0 | 477,4 | in NaCl-Spüllösung: 0,9 µg |

## Patentansprüche

1. Verfahren zum Lösen einer mit Hilfe eines Linkers an eine Polymeroberfläche gebundene Substanz von dieser Oberfläche, wobei die Polymeroberfläche Carbonylgruppen umfaßt und der Linker mindestens eine zur Wasserstoffbrückenbindung fähige funktionelle Gruppe aufweist, **dadurch gekennzeichnet, daß** die Bindung des Linkers an der Oberfläche durch Zugabe eines polaren organischen Lösungsmittels ausgewählt aus Alkanolen und Estern getrennt wird.

2. Verfahren nach Anspruch 1, wobei das Lösungsmittel Methanol ist.

3. Verfahren nach einem der Ansprüche 1 oder 2, wobei das Lösungsmittel in Form einer wäßrigen Lösung mit einer Konzentration von maximal 60 Vol.-% eingesetzt wird.

4. Verfahren gemäß einem der Ansprüche 1 bis 3, wobei die Polymeroberfläche mindestens ein Strukturelement (A) umfaßt, 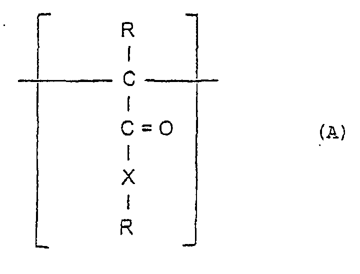 in der die Reste R gleich oder verschieden sein können und einen Alkyl- oder Arylrest oder ein Wasserstoffatom darstellen und in der der Rest X eine Bindung, O, N oder CH₂ darstellt, wobei im Fall X=N ein zusätzlicher, wie vorstehend definierter Rest R mit X verbunden ist.

## Claims

1. Process for separating a substance, which is bonded to a polymer surface via a linker, from said surface, the polymer surface comprising carbonyl groups and the linker having at least one functional group capable of forming a hydrogen bond, **characterized in that** the bond between the linker and the surface is separated by adding a polar organic solvent selected from alkanols and esters.

2. Process according to claim 1, wherein the solvent is methanol.

3. Process according to any of claims 1 or 2, wherein the solvent is used in the form of an aqueous solution in a concentration of not more than 60% by volume.

4. Process according to any of claims 1 to 3, wherein the polymer surface comprises at least one structural element (A) 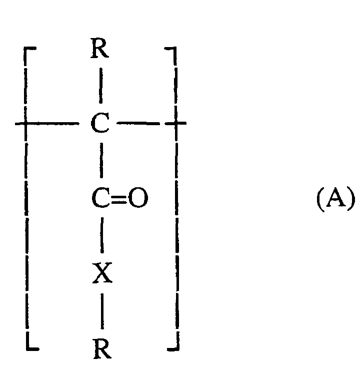 wherein the residues R may be equal or different and represent an alkyl or aryl group or a hydrogen atom, and in which the residue X represents a bond, O, N or CH₂, wherein in the case X=N an additional residue R as defined above is bonded with X.

## Revendications

1. Procédé de dissolution, depuis une surface de polymère, d'une substance liée par un linier à cette surface, la surface de polymère comprenant des groupes carbonyle et le linker présentant au moins un groupe fonctionnel capable de former des ponts hydrogène, **caractérisé en ce que** la liaison du linker sur la surface est séparée en ajoutant un solvant organique polaire choisi parmi les alcanols et les esters.

2. Procédé selon la revendication 1, dans lequel le solvant est du méthanol.

3. Procédé selon l'une ou l'autre des revendications 1 et 2, dans lequel le solvant est utilisé sous forme d'une solution aqueuse dans une concentration de 60 % en volume au maximum.

4. Procédé selon l'une des revendications 1 à 3, dans lequel la surface du polymère comprend au moins un élément de structure (A) 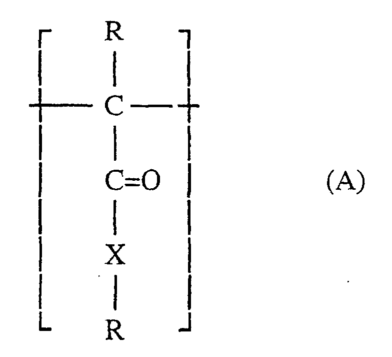 dans lequel les restes R peuvent être égaux ou différents et constituent un reste d'alkyle ou d'aryle ou un atome d'hydrogène et dans lequel le reste X constitue une liaison, O, N, ou CH₂ et dans le cas X=N, un reste R additionnel, tel que défini ci-dessus, est relié à X.
